# EUROPÄISCHE PATENTSCHRIFT

(11) **EP 1 802 989 B1**
(45) Veröffentlichungstag und Bekanntmachung des Hinweises auf die Patenterteilung: **20.04.2011**
(21) Anmeldenummer: 05807931.0
(22) Anmeldetag: 21.10.2005
(51) Int. Cl.: G01R 33/563

(54) **KONTINUIERLICHES GEFÄSS-SELEKTIVES SPIN LABELING**
CONTINUOUS VESSEL-SELECTIVE SPIN LABELING
MARQUAGE CONTINU PAR SPIN, SELECTIF DE VAISSEAU

(30) Priorität: 23.10.2004 DE 102004051763
(43) Veröffentlichungstag der Anmeldung: 04.07.2007
(73) Patentinhaber: Universitätsklinikum Schleswig-Holstein, 24105 Kiel (DE)
(72) Erfinder: WERNER, Richard, 20253 Hamburg (DE); NORRIS, David, G., NL-6581 NJ Malden (NL)
(74) Vertreter: Biehl, Christian
(86) Internationale Anmeldenummer: PCT/DE2005/001891
(87) Internationale Veröffentlichungsnummer: WO 2006/042536

(56) Entgegenhaltungen:
- WO-A-90/12329
- US-A- 5 402 785
- US-A1- 2003 193 334
- WERNER R ET AL: "Continuous artery-selective spin labeling (CASSL)" MAGNETIC RESONANCE IN MEDICINE, Bd. 53, 20. April 2005 (2005-04-20), Seiten 1006-1012, XP002364002
- EASTWOOD JD ET AL: "Magnetic resonance imaging with lateralized arterial spin labeling" MAGNETIC RESONANCE IMAGING, Bd. 20, 2002, Seiten 583-586, XP002364003
- ZAHARCHUK G ET AL: "Multislice perfusion and perfusion territory imaging in humans with separate label and image coils" MAGNETIC RESONANCE IN MEDICINE, ACADEMIC PRESS, DULUTH, MN, US, Bd. 41, Nr. 6, Juni 1999 (1999-06), Seiten 1093-1098, XP002231755 ISSN: 0740-3194
- HINSHAW W S: "IMAGE FORMATION BY NUCLEAR MAGNETIC RESONANCE: THE SENSITIVE-POINT METHOD" JOURNAL OF APPLIED PHYSICS, AMERICAN INSTITUTE OF PHYSICS. NEW YORK, US, Bd. 47, Nr. 8, August 1976 (1976-08), Seiten 3709-3721, XP001182859 ISSN: 0021-8979

## Beschreibung

Die Erfindung betrifft ein Verfahren zur MRT-Darstellung eines Blutgefäßes und/oder des von dem Blutgefäß versorgten Territoriums durch Markieren des von dem Blutgefäß geführten Bluts mittels kontinuierlichem Spin-Labeling (continuous arterial spin-labeling: CASL) in einer bestimmten Markierungsebene.

Zur Darstellung eines Blutgefäßes und/oder des von dem Blutgefäß versorgten Territoriums sind bereits mehrere Verfahren entwickelt worden, die eine Aussage über die arterielle Blut- und Sauerstoffversorgung von dem Blutgefäß versorgter Territorien ermöglichen. Diese Verfahren sind insbesondere bei der-Darstellung zerebraler Perfusionsterritorien von Interesse, da die individuelle Durchblutungssituation des Gehirns als eine zentrale neuroradiologische Fragestellung aufgrund zahlreicher Faktoren von der Normalsituation, die auf Basis histologischer Befunde bekannt ist, abweichen kann. Im Folgenden soll deshalb beispielhaft auf die Anwendung solcher Verfahren in der Neuroradiologie Bezug genommen werden.

Eine in der klinischen Praxis weit verbreitete Methode ist die Digitale Subtraktions-Angiographie (DSA). Durch Einspritzen eines Kontrastmittels werden die Gefäßbäume der großen, das Gehirn versorgenden Arterien visualisiert, wobei durch Vorschieben eines Katheters auch die Gefäßbäume kleinerer Arterien abgebildet werden können. Der Nachteil dieser Methode besteht darin, dass es sich um ein invasives Verfahren handelt, dessen Durchführung besonders bei Patienten mit cerebrovaskulären Krankheiten mit einem hohen Risiko verbunden sein kann. Ein weiterer Nachteil ist, dass die Injektion des Kontrastmittels zu Veränderungen der natürlichen Druckverhältnisse und damit zu abweichenden Ergebnissen gegenüber der tatsächlichen Situation führen kann. Außerdem können nur die Gefäße bis zu einer bestimmten Mindestgröße dargestellt werden. Das Verfahren gibt somit keinen Aufschluss über die tatsächliche Durchblutung des Gewebes (Perfusion), da die dafür verantwortlichen Kapillargefäße nicht erfasst werden können.

Eine mögliche Alternative bieten nicht-invasive Verfahren, bei denen das die das Gehirn versorgenden Arterien und die Gehirnareale durchströmende Blut selbst als intrinsisches Kontrastmittel verwendet wird. Dieses Verfahren wird als arterielles Spin-Labeling (ASL) bezeichnet.

Zur Bestimmung der zerebralen Blutzirkulation werden hierzu ein Markierungs- und ein Kontrollexperiment durchgeführt. Beim Markierungsexperiment wird die Magnetisierung des Blutes in den das Gehirn mit Blut und Sauerstoff versorgenden Arterien mit geeigneten Pulssequenzen invertiert. In einer sich an die Markierung anschließende Phase wird das in das Gehirn strömende Blut mit einem Bildgebungsverfahren (z.B. echo planar imaging: EPI) detektiert. Im Kontrollexperiment hingegen werden Pulssequenzen angewendet, die die Magnetisierung des Blutes im Idealfall gar nicht verändern. Dabei ist Voraussetzung, dass die im Kontrollexperiment verwendeten Pulse denselben Sättigungseffekt wie die während der Markierung verwendeten Pulse besitzen. Dadurch wird gewährleistet, dass die Bilder des Markierungs- und des Kontrollexperiments, mit Ausnahme des unterschiedlichen Magnetisierungszustands des Bluts, identisch sind und die zerebralen Perfusionsterritorien durch Subtraktion der jeweils zueinander gehörenden Bilder sichtbar gemacht werden können.

Je nach Art der verwendeten Pulse wird das arterielle Spin-Labeling in zwei Verfahren unterteilt. Beim gepulsten ASL-Verfahren (PASL) werden kurze Inversionspulse verwender, die den Magnetisierungszustand des Blutes in einer dicken Schicht proximal des zu untersuchenden Bereichs invertieren, wobei das markierte Blut nach der erfolgten Inversion in das Gewebe fließt. Beim kontinuierlichen ASL-Verfahren (CASL) hingegen werden kontinuierliche RF-Pulse mit einer Länge von ca. 2 s verwendet, die eine adiabatische Inversion des Magnetisierungszustands des in das Gewebe einströmenden Blutes hervorrufen. Ein häufig angewendetes CASL-Verfahren mit einem amplitudenmodulierten Kontrollexperiment findet sich bei DC ALSOP und JA DETRE ("Multisection cerebral blood flow MR imaging with continuous arterial spin labeling" (1998) Radiology 208(2):410-6).

Aus der Druckschrift WO 90/12329 A1 ist ein MRT-Verfahren zur Selektion eines Volumenbereichs bekannt. Bei dem bekannten Verfahren werden magnetische Gradientenfelder derart mit breitbandigen RF-Pulsen kombiniert, dass es entlang der Gradientenrichtung mit Ausnahme eines zentralen Bereichs, in dem das Spektrum des RF-Pulses eine Lücke aufweist, zu einer magnetischen Sättigung des Gewebes kommt.

Aus der Druckschrift US 2003/0193334 A1 ist ein kontinuierliches Spin-Labeling-Verfahren mit den Merkmalen des Oberbegriffs des Anspruchs 1 bekannt. Bei dem bekannten Verfahren verbleibt die Lage der Markierungsebene in einer festen Position.

Der Nachteil der herkömmlichen ASL-Verfahren besteht darin, dass die Darstellung von einem einzelnen Blutgefäß zugeordneten Territorien nur in wenigen Einzelfällen möglich ist. Die räumliche Anordnung des Blutgefäßsystems und die räumliche Auflösung, die mit dem bekannten Verfahren erreicht werden kann, führen deshalb im Wesentlichen immer zu einer Darstellung mehrerer Territorien, die auf mehrere Blutgefäße zurückzuführen sind.

Aufgabe der Erfindung ist es deshalb, ein Verfahren zur MRT-Darstellung eines Blutgefäßes und/oder des von dem Blutgefäß versorgten Territoriums durch Markieren des von dem Blutgefäß geführten Bluts mittels kontinuierlichem Spin-Labeling zu schaffen, das eine genauere Markierung einer einzelnen Arterie und damit eine Darstellung eines von einer einzelnen Arterie versorgten Perfusionsterritoriums ermöglicht.

Die Aufgabe wird gelöst durch ein Verfahren zur MRT-Darstellung eines Blutgefäßes und/oder des von dem Blutgefäß versorgten Territoriums gemäß Anspruch 1.

Das erfindungsgemäße Verfahren nutzt ein Markieren zur MRT-Darstellung eines Blutgefäßes (A) und/oder des von dem Blutgefäß (A) versorgten Territoriums mittels kontinuierlichem Spin-Labeling in einer bestimmten Markierungsebene (E), wobei die Lage der Markierungsebene (E) unter der Maßgabe, dass ein bestimmter Ort des Blutgefäßes (A) in der Markierungsebene (E) verbleibt, variiert wird. Dies kann zum Beispiel ein periodisches Variieren der Markierungsebene (E) und/oder stufenweises Variieren der Markierungsebene (E) während der Markierung bedeuten. Die Lage der Markierungsebene kann aber auch in nichtperiodischer Weise variiert werden.

Mit dem Verfahren kann ein Blutgefäß und/oder das von dem Blutgefäß versorgte Territorium dargestellt werden. Dazu zählt sowohl das markierte Blutgefäß selbst als auch der sich an dieses Gefäß distal anschließende Gefäßbaum. Dadurch können Angiogramme von Gefäßen oder Gefäßbäumen erstellt werden. Weiterhin ist es vorgesehen, auch die Durchblutung des von dem markierten Gefäß versorgten Gewebes, des Perfusionsterritoriums des Gefäßes, darzustellen. Neben der im Ausführungsbeispiel beispielhaft gezeigten Anwendung zur Darstellung zerebraler Perfusionsterntorien ist das Verfahren grundsätzlich auch zur Darstellung von Gefäßen, Gefäßbäumen und/oder Perfusionsterritorien in beliebigen anderen Organen, wie z. B: den Nieren, dem Herzen, der Lunge, der Prostata oder in Muskeln des Bewegungsapparats geeignet. Das Verfahren kann auch zur Aufklärung der Bedeutung eines bestimmten Blutgefäßes für die Versorgung von Pathologien wie z.B. Fisteln, Aneurysmen oder Tumoren beitragen. Das Verfahren kann sowohl zur Markierung von arteriellem als auch von venösem Blut herangezogen werden.

Durch das Variieren der Lage der Markierungsebene in der in Anspruch 1 angegebenen Weise werden drei Effekte zugleich erzielt: Erstens ist die Beeinflussung der Magnetisierung während des Markierungs- und Kontrollexperiements im Bereich des stationären Punktes der Marlcierungsebene, der mit dem zu markierenden Blutgefäß zusammenfällt, nahezu identisch mit der im Falle einer ruhenden Markierungsebene erzielten Beeinflussung der Magnetisierung. Dies bedeutet, dass die Markierung in diesem Bereich wirksam wird und ähnlich effektiv ist wie bei einer nicht-selektiven Markierung des durch eine ruhende Ebene strömenden Blutes.

Zweitens kommt es in einem Abstand von dem stationären Punkt der Ebene sowohl im Markierungs- als auch im Kontrollexperiment zu einer Sättigung der Magnetisierung. Dieser Effekt beruht auf einer Pseudo-Zufallsverteilung der Magnetisierung einzelner Spin-Ensembles, die wiederholt durch die sich bewegende Ebene hindurchtreten.

Drittens wird erreicht, dass der Einfluß der während des Markierungs- und Kontrollexperiments verwendeten Pulssequenzen auf die Magnetisierung im Bereich des Bildgebungsvolumens im Markierungs- und Kontrollexperiment identisch ist. Die sogenannten off-resonance-Effekte werden dadurch kompensiert.

Gemäß einer Ausgestaltung wird die Lage der Markierungsebene periodisch variiert. Dies bietet den Vorteil, dass die Pulssequenz für das Markierungs- und Kontrollexperiment ebenfalls periodisch ist und die zeitlich zu variierenden Gradientenfelder und die Amplituden- und Frequenz-Modulation der RF-Pulse nur für eine Periode berechnet werden müssen. Die für eine Periode berechnete Sequenz kann dann entsprechend wiederholt werden.

Gemäß einer Ausgestaltung wird die Lage der Markierungsebene stufenweise variiert. Dadurch kann eine besonders einfache Pulssequenz verwirklicht werden, die nicht auf die Verwendung zeitlich kontinuierlich variierender Felder angewiesen ist. Stattdessen werden die Gradienten und RF-Felder stufenweise geschaltet. Beispielsweise kann die Größe der Stufen so klein gehalten werden, dass sich ein näherungsweise kontinuierliches Variieren der Lage der Markierungsebene erzielt wird. Es lassen sich aber auch bei Verwendung größerer Stufen brauchbare Ergebnisse erzielen.

Bevorzugt kann durch Wahl einer mit einem Neigungswinkel θ zur Längsachse des Blutgefäβes (A) geneigten Markierungsebene (E) und Rotieren der geneigten Markierungsebene (E) um das Blutgefäß (A) als Längsachse, bevorzugt mit konstantem Neigungswinkel aber auch mit variierendem Neigungswinkel eine Darstellung erreicht werden.

Die Größe des Neigungswinkels θ und der Frequenz der Rotationsbewegung ist dabei in weiten Bereichen frei wählbar. Der Neigungswinkels θ kann beispielsweise Werte zwischen 1 und 60 Grad annehmen, bevorzugt Werte zwischen 5 und 25 Grad. Für die Frequenz der Rotationsbewegung können beispielsweise Werte im Bereich von 5 Hz bis 1000 Hz, bevorzugt 40 Hz bis 300 Hz, verwendet werden. Für eine erfolgreiche selektive Markierung kommt es auf eine geeignete Kombination der beiden Parameter an. Eine Rolle spielt hier auch eine Abstimmung auf die Strömungsgeschwindigkeit des Blutes, die sich je nach Anwendung unterscheiden kann.

Die Erfindung wird anhand eines bevorzugten Ausführungsbeispiels mit Bezug auf die beigefügten Zeichnungen näher erläutert. Es zeigen:
- Fig. 1: eine schematische Darstellung der räumlichen Verhältnisse zwischen der Mar- kierungsebene und der zu markierenden Arterie,
- Fig. 2: eine MRT-Darstellung eines Markierungs- und eines Kontrollexperiments und der Subtratkionsdarstellung beider an einem Phantom durchgeführten Experi- mente und
- Fig. 3: eine invertierte Darstellung einer MRT-Darstellung zerebraler Perfusionsterri- torien durchgeführt mit dem erfindungsgemäßen Verfahren.

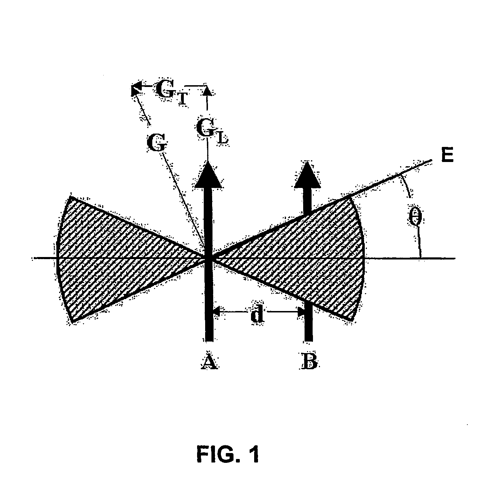

Fig. 1 zeigt die räumlichen Verhältnisse zwischen der Markierungsebene E und der, zur Darstellung eines Blutgefäßes und/oder eines von einem Blutgefäß versorgten Territoriums, das zu markierende Blut führende Blutgefäß A. Das Blutgefäß kann Jede Art von Blutgefäß sein. Regelmäßig wird das Blutgefäß aber eine Arterie sein, wobei bei einer bevorzugten Anwendung in der Neuroradiologie das darzustellende Territorium ein von einer Arterie versorgtes, zerebrales Perfusionsterritorium ist.

Die Markierungsebene E wird derart variiert, dass ein bestimmter Ort des Blutgefäßes A in der Markierungsebene E verbleibt. Das Variieren der Markierungsebene E kann in einem Kippen, Neigen oder auch Rotieren um einen Ort des Blutgefäßes, bevorzugt ein Punkt, höchstens aber ein begrenzter Abschnitt, bestehen, wobei der Ort des Blutgefäßes in der Markierungsebene verbleibt.

In dem in Fig. 1 dargestellten bevorzugten Ausführungsbeispiel wird die Markierungsebene **E** so zur Längsachse des Blutgefäßes **A** gelegt, dass die Markierungsebene **E** im Verhältnis zur Längsachse des Blutgefäßes **A** in einem besonders bevorzugt konstanten Winkel θ geneigt ist. Zur Markierung des das Blutgefäß **A** durchströmenden Blutes rotiert die Markierungsebene **E** mit einer Frequenz fᵣₒₜ um das Blutgefäß **A** als Längsachse.

Die Frequenz des Markierungs-RF-Pulses wird so gesteuert, dass sie immer der Resonanzfrequenz an der Position an dem Blutgefäß **A** entspricht, an der das Blut markiert wird. Die Markierungsebene **E** wird derart variiert, dass ein bestimmter Ort des Blutgefäßes in der Markierungsebene **E** verbleibt.

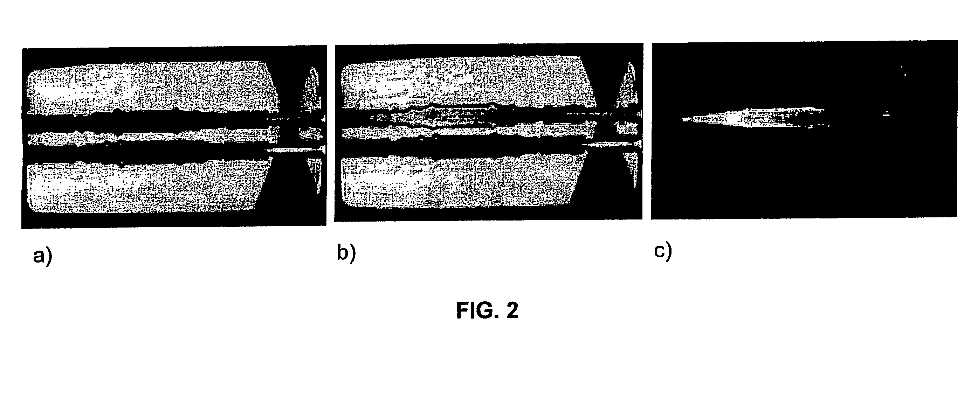

Fig. 2 zeigt die Ergebnisse eines mit dem erfindungsgemäßen Verfahren an einem Phantom durchgefiihrten Markierungs- (a) und eines Kontrollexperiments (b). Die in den Fig. 2a und 2b dargestellten dunklen Kegel sind Artefakte, die an der Stelle auftreten, an der die rotierende Markierungsebene die Bildebene schneidet. Fig. 2c zeigt das Subtraktionsbild aus dem Markierungs- und Kontrollexperiment. Das Phantom bestand aus zwei parallelen Röhren mit einem inneren Durchmesser von 6 mm, die in einem Abstand von 20 mm in Agarosegel eingebettet waren. Jede Röhre war an je einen Schlauchverbinder aus Glas angeschlossen, der an seinen Enden einen Durchmesser von 6 mm hatte, der sich zur Mitte auf ungefähr 12 mm verbreiterte. Daraus ergab sich, dass die Fließgeschwindigkeit des verwendeten Wassers in den dünneren Abschnitten, an denen die Markierung vorgenommen wurde, auf physiologische Werte eingestellt werden konnte, während die Geschwindigkeit in den dickeren Abschnitten merklich reduziert war und dadurch die Messung der Magnetisierung in diesem Bereich vereinfacht wurde. Die für das dargestellte Experiment verwendeten Parameter waren: fᵣₒₜ = 80 Hz; θ = 20° und v = 35 cm/s.

Durch die Relativbewegung von Markierungsebene und nicht zu markierendem Blutgefäß in einem Abstand d von dem zu markierenden Blutgefäß wird erreicht, dass der Markierungsmechanismus nicht wirksam wird. Stattdessen kommt es an der Position des nicht zu markierenden Blutgefäßes zu einer Sättigung der Magnetisierung des fließenden Blutes. Dies gilt für das Markierungs- und für das Kontrollexperiment, so dass das Blut im Subtraktionsbild nicht sichtbar wird.

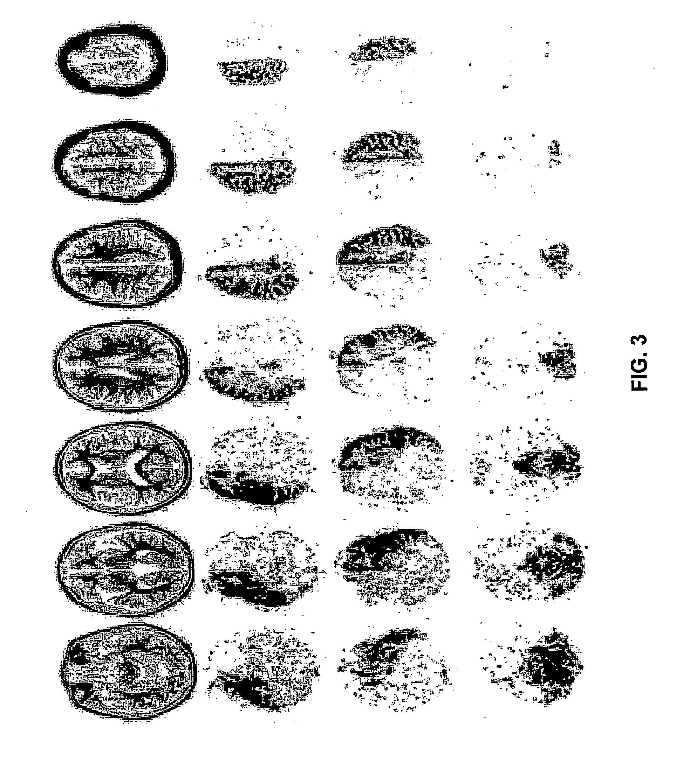

Fig. 3 zeigt eine invertierte Darstellung einer mit dem erfindungsgemäßen Verfahren durchgeführten MRT-Darstellung zerebraler Perfusionsterritorien. Die obere Reihe enthält zur Zuweisung der Schnittebenen anatomische Ansichten als Referenz. Die zweite Reihe enthält Ansichten der Perfusionsterritorien für die rechte Arteria carotis interna. Die dritte Reihe enthält Ansichten der Perfusionsterritorien für die linke Arteria carotis interna und die vierte Reihe enthält Ansichten der Perfusionsterritorien für die Arteria basalis. Jeder Scan dauerte 5 min, wobei sieben Schichten mit 9 mm Dicke mit einem spin-echo echo planar Bildgebungsverfahren (SE-EPI) erstellt wurden.

Fig. 3 zeigt am Beispiel zerebraler Perfusionsterritorien, dass das hier vorgestellte erfmdungsgemäße Verfahren eine selektive Markierung von einzelnen, räumlich eng beieinander liegenden Blutgefäßen ermöglicht. Mithilfe dieses Verfahrens können beispielsweise die Perfusionsterritorien der linken und rechten Arteria carotis interna und der Arteria basilaris voneinander getrennt dargestellt werden.

## Patentansprüche

1. Ein continuous arterial spin labeling Verfahren zur MRT-Darstellung eines Blutgefäßes
(A) und/oder des von dem Blutgefäß (A) versorgten Territoriums wobei
ein Markierungs- und ein Kontrollexperiment durchgeführt werden, wobei
die Magnetisierung des von dem Blutgefäß (A) geführten Bluts im Markierungsexperiment beim Durchströmen einer Markierungsebene (E) durch kontinuierliche RF-Pulse adiabatisch invertiert wird,
**gekennzeichnet durch**
Variieren der Lage der Markierungsebene (E) während der adiabatischen Inversion unter der Maßgabe, dass ein bestimmter Ort des Blutgefäßes (A) in der Markierungsebene (E) verbleibt.

2. Verfahren nach Anspruch 1, **gekennzeichnet durch** periodisches Variieren der Lage der Markierungsebene (E) während der adiabatischen Inversion.

3. Verfahren nach Anspruch 1 oder 2, **gekennzeichnet durch** stufenweises Variieren der Lage der Markierungsebene (E) während der adiabatischen Inversion.

4. Verfahren nach einem der vorangehenden Ansprüche, **gekennzeichnet durch** Wahl einer mit einem Neigungswinkel θ zu einer Längsachse des Blutgefäßes (A) geneigten Markierungsebene (E) und Rotieren der geneigten Markierungsebene um das Blutgefäß (A) als Längsachse während der adiabatischen Inversion.

5. Verfahren nach Anspruch 4, **dadurch gekennzeichnet, dass** der Neigungswinkel θ während der adiabatischen Inversion konstant ist.

6. Verfahren nach einem der vorangehenden Ansprüche, **dadurch gekennzeichnet, dass** das Territorium ein zerebrales Perfusionsterritorium ist.

## Claims

1. A continuous arterial spin labeling method for MRT imaging of a blood vessel (A) and/or the territory supplied by the blood vessel (A),
a marking and control experiment being carried out,
the magnetization of the blood carried in the blood vessel (A) when flowing through a marking plane (E) being adiabatically inverted by continuous HF pulses during the course of the marking experiment,
**characterized by**
varying the position of the marking plane (E) during the adiabatic inversion with the proviso that a certain location of the blood vessel (A) remains in the marking plane (E).

2. The method according to Claim 1, **characterized by** periodically varying the position of the marking plane (E) during the adiabatic inversion.

3. The method according to Claim 1 or 2, **characterized by** stepwise varying the position of the marking plane (E) during the adiabatic inversion.

4. The method according to one of the preceding claims, **characterized by** selecting a marking plane (E) inclined at an angle θ of inclination relative to a longitudinal axis of the blood vessel (A) and rotating the inclined marking plane about the blood vessel (A) as longitudinal axis during the adiabatic inversion.

5. The method according to Claim 4, **characterized in that** the angle θ of inclination is constant during the adiabatic inversion.

6. The method according to one of the preceding claims, **characterized in that** the territory is a cerebral perfusion territory.

## Revendications

1. Un procédé de marquage de spin artériel continue (continuous arterial spin labeling) pour la visualisation par IRM du territoire irrigué par un vaisseau sanguin (A) et/ou par le
vaisseau sanguin (B) avec
l'exécution d'une expérience de marquage et d'une expérience de contrôle, au cours desquelles
la magnétisation du sang convoyé dans le vaisseau sanguin (A) dans l'expérience de marquage est inversée adiabatiquement par des impulsions RF continues lorsque le sang traverse le plan de marquage (E),
**caractérisé par**
la variation de la position du plan de marquage (E) pendant l'inversion adiabatique avec la condition qu'un certain endroit du vaisseau sanguin (A) reste dans le plan de marquage (E).

2. Procédé selon revendication 1, **caractérisé par** une variation périodique de la position du plan de marquage (E) pendant l'inversion adiabatique.

3. Procédé selon revendication 1 ou 2, **caractérisé par** une variation par étapes de la position du plan de marquage (E) pendant l'inversion adiabatique.

4. Procédé selon une des revendications ci-dessus, **caractérisé par** le choix d'un plan de marquage (E) incliné d'un angle d'inclinaison θ par rapport à un axe longitudinale du vaisseau sanguin (A) et la rotation du plan de marquage incliné autour du vaisseau sanguin (A) en tant qu'axe longitudinale pendant l'inversion adiabatique.

5. Procédé selon revendication 4, **caractérisé par le fait que** l'angle d'inclinaison θ est constant pendant l'inversion adiabatique.

6. Procédé selon une des revendications ci-dessus, **caractérisé par le fait que** le territoire est un territoire de perfusion cérébrale.
